# EUROPEAN PATENT APPLICATION

(11) **EP 4 628 325 A1**
(43) Date of publication of application: **08.10.2025**
(21) Application number: 25167204.4
(22) Date of filing: 28.03.2025
(51) Int. Cl.: B60C 1/00, C08K 5/101, C08L 9/00, C08L 9/02, C08L 9/06, C08L 7/00, C08K 5/00

(54) **ESTERS FROM VEGETABLE OILS AS PROCESS OILS FOR THE PRODUCTION OF ELASTOMERS**

(30) Priority: 04.04.2024 IT 202400007495
(71) Applicant: Fluos S.A.S. Di Giuseppe Chiaradia & C., 21052 Busto Arsizio (VA) (IT); Chemcom Investments BV, 9936 HD Farmsum (NL)
(72) Inventor: CHIARADIA, Giuseppe, Torino (IT); CATALDO, Franco, Roma (IT); BASTIAENSEN, Erik, Kapellen (BE); COLOMBO, Dario, Busto Arsizio (VA) (IT); TURESSO, Caterina, Busto Arsizio (VA) (IT)
(74) Representative: Giugni, Diego

(57) **Abstract**

The present invention concerns the use of specific fatty acid esters for the production of elastomers, in particular in the production of tyre treads, which are given better performance properties. In addition, the invention relates to elastomers comprising these fatty acid esters, as well as tyres or tyre treads comprising such elastomers.

## Description

### TECHNICAL FIELD OF THE INVENTION

The present invention concerns the use of fatty acid esters of Glycerol Formal as process oils in the production of elastomers, in particular in the production of tire treads.

### BACKGROUND OF THE INVENTION

The most widespread use of elastomers is in the construction of automotive tires. Other applications are in the automotive industry, household appliances, building construction, textiles and biomedical applications.

Elastomers (elastomeric polymers) are distinguished from plastomers (plastic polymers, so-called plastics) on the basis of the behaviour of suitable specimens subjected to stretching. While elastomers give rise to considerable deformations under relatively modest stresses, plastic polymers undergo much lower elongation even when the stress reaches the tensile strength. From a functional point of view, plastic polymers are characterized by their flexibility, while elastomers are characterized by their elasticity.

The main components of compounds for the production of synthetic rubbers are elastomeric polymers, reinforcing fillers, process oils (also named: rubber process oils, extenders, plasticizers), vulcanization agents. Other additives, such as stabilizers and antioxidants are included in smaller amounts.

Reinforcing fillers are incorporated into the compositions for the production of synthetic rubbers in order to increase rigidity and mechanical strength. In the past, Carbon Black was widely used in the production of tires, but in recent years carbon black has been largely replaced by precipitated silica. However, fillers increase viscosity during processing, which leads to serious complications in the rubber manufacturing process that necessitate the addition of a thinner.

Process oils are mainly added to improve the dispersibility of the *filler* and reduce the viscosity of the compound, thus allowing not only to have a homogeneous distribution of the *filler* and other components, but also a reduction in the tangles of the long polymer chains, thus allowing to improve the distensibility characteristics of the chains. For this property of untangling the tangles between the polymer chains, process oils are also called extenders. In addition, a more correct distribution of chains allows to optimize the process of vulcanization or cross-linking with sulphur of the double bonds contained in the polymer chains of the elastomer.

Aromatic petroleum oils are widely used as process oils due to their compatibility with styrene butadiene (SBR) and other unsaturated polydienes rubbers. The most common are residual aromatic extract (RAE), treated distilled aromatic extract (TDAE), mild or medium solvate extract (MES). Naphthenic oils are also used

Since the nitrile elastomers have a higher polarity than styrenic elastomers, the use of phthalate-based plasticizers such as dioctyl phthalate (DOP) as an alternative to aromatic petroleum oils, due to their greater polarity, is widespread. However, in recent years there has been a tendency to replace phthalates with carboxyl diesters, as they are the known endocrine disrupting phthalates that have been proven to be linked to obesity, insulin resistance, asthma and attention deficit hyperactivity disorder. Some diesters that are most frequently used are dioctyl adipate and dibutyl sebacate.

Under the Regulation of the European Parliament and of the Council on REACH (EC 18/09/2006), highly aromatic oils containing polycyclic aromatic hydrocarbons (PAHs) have been banned as of 1 January 2010. The limits of PAH in the process oil shall be less than 3 % by mass, measured according to the IP346 method. Manufacturers of rubber process oils have offered some environmentally friendly alternative process oils with low PAH content such as TDEA and MES. However, few studies have been published so far on the possible adverse effects of oils with a low aromatic content

Of particular relevance is the problem of the dispersion of elastomeric particles in the environment, due to the abrasion of the tread of motor vehicle tires on the asphalt. According to Mordor Intelligence's website, 3.52 million tons of rubber processing oil were produced in 2023, and production is expected to reach 4.07 million tons in 2028. However, almost all of the tyre's plasticisers are found in the tread, which wears out over time due to the abrasion produced by friction between the rolling tyre and the asphalt. Since the tyre is replaced after almost all of the tread has worn out, it can be assumed that at least 50% of the oil in petroleum plasticisers is dispersed into the environment.

It should also be borne in mind that the molecules of petroleum oils, having an aromatic structure, are insoluble in water and are difficult to biodegrade, and consequently the dust produced by abrasion tends to accumulate in the environment over the years. If we then consider that oils generally make up about 15% by weight of the tread, the amount of dust emitted each year due to tire wear is probably more than 20 million tons per year. In addition, the finest particles that settle on the asphalt are easily lifted by car traffic and thus form the so-called fine particles that pollute large cities.

Therefore, due to the health and environmental problems caused by aromatic oils used as process oils, there is a growing demand for alternative oils that are renewable, safe, sustainable and environmentally friendly.

As an alternative of renewable origin to fossil processing oils, patent literature mainly reports soybean, sunflower and palm vegetable oils as a partial or total replacement for aromatic oils.

*Process oils* based on vegetable oils used commercially in the production of tire treads are added in order to increase flexibility at low temperatures. However vegetable oils can generally be added in modest quantities, only partially replacing aromatic oils, or, in the case of total replacement, the use of other adjuvants is required so that the required performance is conferred.

For example, patent application US2014/0135437 proposes the use of soybean oil in combination with certain resins. As for esters, EP 3251872 A1 describes some fatty acid esters that lower the glass transition temperature and therefore the performance of elastomers in winter.

However, vegetable oils and carboxylic esters have low compatibility with the diene polymers that make up most commercially used elastomers. It follows that if the concentration of the oil or ester in the composition exceeds the limits of compatibility, there is an exudation of the oil molecules towards the outer surface of the elastomer, evidenced by the sticky surface on contact with the fingers and the greasy appearance. Over time, the continuous migration of the ester or oil leads to an irreversible loss of the elastic properties of the material.

With reference to the use of elastomers in the biomedical applications, in particular medical gloves and condoms, it is known that by virtue of its molecular structure, natural rubber latex is a crossed-linked polymeric material that is highly flexible and extensible. It should be noted that the molecular structure that provides flexibility to the material inherently and necessarily imparts the characteristic of permeability to some substances. The flexibility is derived from a degree of molecular mobility, which implies an ability for certain substances, depending on atomic or molecular size and affinity with the latex components to pass through this molecular structure. This process of diffusion is naturally time-dependent. Most procedures that depend upon the latex barrier function are of sufficiently short duration for this diffusion process to be of no practical consequence. Of some clinical importance, however, is the fact that contact with certain chemicals may facilitate this process of diffusion through swelling or other processes.

Accordingly, currently, plasticizer is not used in the production of nitrile or latex gloves and in the production of condoms.

### SUMMARY OF THE INVENTION

A first object of the present invention is the use of esters obtained from the esterification of C₆-C₂₂ fatty acids with Glycerol Formal (acronym GFE) as process oils in the production of elastomeric materials such as styrene butadiene rubbers (SBR), nitrile rubbers (NBR), polyurethane and acrylic elastomers, polyesters etc., able to improve the dispersion of compound components during the production of synthetic rubber and to improve the characteristics of elasticity of the finished material.

A second object of the present invention is the use of these esters in the production of tyre treads in order to improve road performance at low temperatures or, by varying the cross-linking density, to reduce tread abrasion.

A third object of the present invention is the use of these esters in the production of medical gloves and condoms made of elastomers such as natural and nitrile rubbers with improved elastic properties that allow to create also a better barrier against microorganisms.

### DETAILED DESCRIPTION OF THE INVENTION

A car tyre must perform well in the following properties: abrasion resistance, rolling resistance, wet grip, maintenance of elasticity at low temperatures (*winter performances*). A simultaneous improvement of all performance by replacing current aromatic oils with other types of processing oils is difficult to achieve, as the properties listed above are interrelated and an improvement in one parameter typically results in a deterioration in the other.

When the tire rolls on the asphalt, a form of friction is created due to rolling. This can be explained by the continuous deformation of the elastomer from the moment the molecules of the polymer chains come into contact with the ground (compression) to the instant they detach from the ground (expansion). Since the compression-expansion mechanism is not perfectly elastic, heat is generated. This loss of energy of the elastomer is called hysteresis.

In order to have good rolling resistance (lower fuel consumption), the elastomer must provide good elastic behaviour and low hysteresis. On the contrary, for good wet performance (increased safety), the elastomer must have a high degree of hysteresis.

It was surprisingly discovered that using esters obtained from the esterification of fatty acids with the Glycerol Formal (named GFE) as an alternative to other extenders, some important performance characteristics of the elastomer were significantly improved without any evidence of ester exudation phenomena on the surface of the elastomer.

As a result of the increased elasticity of the rubber, the use of the tyre tread material allows for better performance in winter driving, where low temperatures cause an increase in the modulus of elasticity of the elastomers.

Among the improvements achieved when using GFE as a process oil, of particular importance is the decrease in the modulus of elasticity of the elastomer compared to traditional petroleum oils in compositions with the same formulation. This means greater elasticity, measured by elongation with the same effort and consequently the use of GFE in the tread of the tires provides better performance in winter driving, where low temperatures cause an increase in the modulus of elasticity of the elastomers, with the consequent loss of flexibility and hardening of the tire. In this way, of course, safety is increased when driving a vehicle in winter periods.

Alternatively, by exploiting the same property of providing elastomers with a lower modulus of elasticity, it is possible to obtain an improvement in the abrasion resistance of the tyre by increasing the amount of vulcanising agents in the production of standard use tyres for use in climates that are not very cold or on roads where there is a possibility of snowfall.

GFE is a fatty acid ester with Glycerol Formal that comes in liquid form with a boiling temperature between 190-195°C and chemically belongs to the lipid class. The graphical representations of the two isomers of the 6- and 5-atoms of dioxolane ring are as follows:

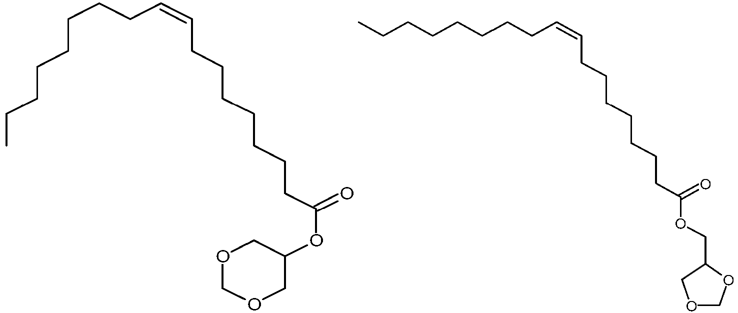

The chemical name is oleic acid, 1,3-dioxan-5-yl ester or oleic acid, (1,3-dioxolan-4-yl) methyl ester or isomer, and the molecular formula is C₂₂H₄O₄

Fatty acids, which make up the acidic part of the GFE ester, are industrially produced by the hydrolysis reaction of vegetable oils, from which the triglyceride components are obtained: glycerin and fatty acids. Table 1 below shows the composition of the main fatty acids in rapeseed, soybean and palm oil, as percentage.

**TABLE 1 - COMPARISON OF THE MAIN FATTY ACIDS IN SOME VEGETABLE OILS**

| Main fatty acids | | Coconut | Rapeseed | Soybean | Palm |
|---|---|---|---|---|---|
| Octanoic | Caprylic (C08:0) | 9,7 | - | - | - |
| Decanoic | Capric (C10:0) | 7,5 | - | - | - |
| Dodecanoic | (Lauric (C12:0) | 42,10 | - | - | - |
| tetradecanoic | Myristic (C14:0) | 22,40 | - | - | - |
| Hexadecanoic | Palmitic (C16:0) | 18,20 | 3,6 | 10,8 | 44,0 |
| Octadecanoic | Stearic (C18:0) | - | 1,5 | 4,0 | 4,5 |
| 9-octadecenoic | Oleic (C18:1) | - | 61,6 | 23,8 | 39,2 |
| 9,12-octadecadienoic | Linoleic (C18:2) | - | 21,7 | 53,3 | 10,1 |
| 9,12,15-octadecatrienoic | Linolenic (C18:3) | - | 9,6 | 7,6 | 0,4 |

Fatty acid esters with Glycerol Formal can be obtained by direct esterification of a fatty acid with Glycerol Formal, or by transesterification of fatty acid methyl ester (acronym: FAME) with Glycerol Formal.

The production methods are those typical of this type of esterification. The method using titanates as a catalyst is conducted at 220°C with a reaction time of 6-8 hours, depending on the excess alcohol used to promote the kinetics of the reaction.

GFE derived from vegetable oils such as soybean, rapeseed, palm, sunflower oil can advantageously replace petroleum-based process oils or others extenders as phthalates in elastomers such as SBR and NBR rubbers, achieving an improvement in the flexibility characteristics of the finished product. In fact, from the experimental data of the stress-strain properties of the SBR and NBR plasticized elastomers, it appears that GFE gives a lower modulus of all elongations than other reference oils.

In SBR rubber, the significantly higher elongation at break measured on GFE compounds confirms the very good elasticizing action on the rubber compound compared to standard MES oil.

A very important application result of this improved performance of GFE compared to petroleum oils is the excellent winter performance in the formulation of vulcanized diene rubber compositions for tire treads when replacing petroleum oils with GFE while maintaining the same dosage.

Despite the lower modulus, GFE is able to maintain the same tensile strength as the reference MES with a higher elongation at break with the same formulation. This allows certain performance targets to be achieved by changing the formulation of the mixture. In particular, by increasing the amount of sulphur and other cross-linking agents in the composition of the tread, the degree of cross-linking of the elastomer can be increased and consequently tyre abrasion can be reduced while maintaining other performance at appropriate values

In NBR rubber applications, the performance of GFE is even better than with plasticizers such as Dibutyl Sebacate or Dioctyl adipate.

For the reasons explained above, medical authorities have established tests to evaluate the protection against penetration of micro-organisms through medical gloves and condoms. In the case of medical gloves in Europe, the ISO166040:2004 standard uses bacteriophage Phi-X174 as the penetration probe. In particular, as will be demonstrated in the Example, the introduction of GFE among the components of the elastomer improves the protection of the user from the penetration of viruses and bacteria.

In accordance with the present invention, it is possible to provide an elastomeric composition for tires in which the amount of elastomers can vary from 20% to 90% wt and the amount of formal esters of C₈-C₁₈ fatty acids of glycerol in an amount between 10% and 80% wt, preferably between 20% and 70% wt of said fatty acids, more preferably 50% wt of fatty acids.

For latex gloves and condoms, preferably, the composition comprises from 1% to 60 % wt. of said esters, more preferably from 5 to 50 %wt, and elastomer from 99% to 40% wt.

### APPLICATION EXAMPLES

The following examples show the results of experimental studies on the performance characteristics of elastomers such as SBR and NBR. The studies were carried out in comparison with some reference plasticizers in their respective fields of application.

### EXAMPLE 1. Tire tread

In this example, the difference in tire performance is compared by replacing a petroleum-based process oil with the GFE ester of the present invention. GFE made from rapeseed oil was used, while MES aromatic oil was chosen as the typical reference oil. To this end, a typical tread composition of passenger car tyres, shown in Table 2, was subjected to experimental tests. This formulation is based on a mixture of elastomers consisting of dry-type styrene butadiene rubber (S-SBR) with 25% styrene and 64% vinyl, a high cis-1,4-polybutadiene obtained with neodymium-based catalyst (Nd-BR) and natural rubber (cis-1,4-polyisoprene) of the SIR-10 type. The fillers are Ultrasil 7000 silica and ASTM N375 standard grade carbon black. The other components are all typical elastomer vulcanization additives with the composition of this example. Relative quantities are expressed in parts by weight per 100 parts of total elastomers (phr). For example, the reference plasticizers, GFE and MES each have a concentration of 34.5 parts referred to 100 parts by weight of total elastomers which are the sum of the parts of S-SBR + Nd-BR + Natural rubber = 102.5 parts. These quantities are represented by the unit of measurement phr (parts hundred rubber).

Furthermore, as per the following experimental data, it has been seen that the glass transition temperature of a tread obtained with the composition of the invention is lower than -70°C and has a viscous modulus at -20°C lower than 11 MPa. Further characteristics are related to the ratio between E"/E' at -20°C which is less than 0.2, the ratio between E"/E' at 0°C which is less than 0.15 and the breaking strength is greater than 11.00 Mpa, after aging, as shown by the experimental data reported below.

**TABLE 2 - RUBBER COMPOSITION FOR TREAD**

| COMPONENT | GFE (PHR) | ESM (phr) |
|---|---|---|
| S-SBR FX5000 Arlanxeo | 52,5 | <------ |
| Nd-BR (Europrene BR40) | 35,0 | <------ |
| Natural rubber | 15,0 | <------ |
| Ultrasil 7000 silica | 65,0 | <------ |
| Carbon Black N375 | 22,0 | <------ |
| Fatty acid ester GFE | 34,5 | |
| Aromatic hydrocarbon MES | | 34,5 |
| Silane Si-69 | 7,0 | <------ |
| ZnO | 4,0 | <------ |
| Stearic acid | 2,0 | <------ |
| Paraffin | 1,0 | <------ |
| 6PPD | 1,5 | <------ |
| CBS | 1,8 | <------ |
| DPG | 1,8 | <------ |
| Sulphur | 1,5 | <------ |
| Total PHR | 244,6 | <------ |

### Preparation of the rubber composition

Two step-by-step methods were used in a 1.5-litre mixer to prepare the composition of the rubber to be tested. In the first phase, elastomers, fillers and plasticizers are introduced into the mixer and the mixture is energetically mixed at temperatures between 150°C and 180°C in order to optimize the distribution of the filler in the mixture of the elastomer chains. In the second step, the elastomer cross-linking reaction is performed. The mixture is cooled to temperatures below 110 °C and then the necessary components for cross-linking are introduced.

### Test methods

The rheometric curves were recorded on an oscillating disc rheometer (ODR) at 175°C for 6 minutes according to the general rules of ISO 3417:2008. The stress-strain properties and final properties of the elastomer specimens were tested according to the ISO 37:2017 test method. Aged stress-strain properties were determined on elastomer specimens aged in the furnace for 3 days at 100°C. The measurement of Shore A Hardness was performed according to ISO 7619-1:2010. The viscoelastic properties were measured on a Mettler-Toledo Star 1 DMA machine that performs Dynamic Mechanical Analysis (DMA) measurements. The measurements were performed in a temperature scan from -100 to +100 °C at a heating rate of 2 °C/min. The Tan δ curve was determined following ASTM E1640-13 (2018) Test Method.

### Torque after cross-linking

Table 3 shows some characteristic data measured by the rheometer during the vulcanization process of the elastomer whose composition is shown in table 2. MH represents the maximum torque reached by the rheometric curve at the end of the cross-linking reaction, while ML is the minimum torque before starting vulcanization.

**TABLE 3. RHEOMETRIC TEST - TORQUE DATA**

| Torque 175°C x 6 min | GFE | MES |
|---|---|---|
| ML (dN m) | 16,62 | 16,42 |
| MH (dN m) | 61,62 | 68,97 |
| MH-ML (dN m) | 45,00 | 52,55 |

ML provides an indication of the viscosity of the uncured rubber compound, and there is no evidence of significant differences between the GFE oil and the reference MES oil. MH is correlated with the modulus of elasticity at the end of crosslinking. MH-ML is correlated with cross-linking density and the lowest value is that of GFE, about 14% less than MES oil.

### Hardness

The hardness of elastomers is measured by the Shore A hardness tester. Higher numbers on the Shore A Hardness scale indicate greater resistance to penetration by a special foot and therefore harder materials. Lower numbers indicate less strength and softer materials. Immediately after vulcanization, GFE gives the same hardness as MES oil. After aging, MES oil shows an increase of +3 Shore A points in contrast to GFE where the increase is limited to +2 points (table 4).

**TABLE 4 - SHORE A HARDNESS**

| Shore A hardness | GFE | MES |
|---|---|---|
| After cross-linking | 71 | 71 |
| Maturation 3 days at 100 °C | 73 | 74 |

### Mechanical properties after cross-linking

Table 5 shows the modulus, tensile strength, and elongation at break after crosslinking.

**TABLE 5 - STRESS-STRAIN PROPERTIES AFTER CROSS-LINKING**

| TENSILE STRENGTH | Unit | GFE | MES |
|---|---|---|---|
| Stress at 50% elongation, | Mpa | 2,36 | 2,57 |
| Stress at 100% elongation | Mpa | 3,76 | 4,41 |
| Stress at 200% elongation | Mpa | 7,17 | 9,01 |
| Stress at 300% elongation | Mpa | 11,17 | 13,36 |
| Tensile strength at break | Mpa | 14,8 | 14,9 |
| Elongation at break | % | 397 | 323 |

In general, compounds with low modulus give greater elongation and lower tensile strength, while rubber with GFE as a process oil shows a high elongation at break, +33% compared to MES, a tensile strength practically equal to that used by MES as a process oil.

### Mechanical properties after aging

Table 6 shows the stress at different degrees of elongation, the stress at break, and the elongation at break after 3-day aging of elastomer specimens at 100°C.

**TABLE 6**

| STRESS-STRAIN PROPERTIES AFTER AGING | | | |
|---|---|---|---|
| Tensile strength | Unit | GFE | MES |
| Effort at 50% elongation, | Mpa | 2,22 | 2,42 |
| Strain at 100% elongation | Mpa | 3,92 | 4,56 |
| Strain at 200% elongation | Mpa | 7,85 | 9,63 |
| Strain at 300% elongation | Mpa | 12,29 | - |
| Stress at break | Mpa | 12,8 | 10,4 |
| Elongation at break | % | 316 | 216 |

After aging, the stress-strain properties for GFE are further improved compared to MES. Experimental results indicate that tensile strength and elongation at break are 23% and 46% higher, respectively, compared to MES.

### Dynamic mechanical properties not aged

Dynamic Mechanical Analysis (DMA) is able to provide a realistic estimate of the main performance required of tread compounds.

The elastic modulus E' (also called accumulation modulus) provides indications on rolling resistance: the higher it is, the lower the elongation of the elastomer for the same effort and therefore there is less consumption of elastic energy during lamination. On the other hand, the viscous modulus E" (also called loss modulus) gives indications of the production of heat when rolling the tire on the asphalt. To achieve good wet grip performance, the tread must have a high hysteresis and therefore a high E."

From the DMA curve from -100°C to +100°C of the tire tread composition, the elastic modulus (E'), the loss modulus (E") and the mechanical hysteresis tan δ (E"/E' ratio) can be extracted. The -20°C elastic modulus is used to evaluate winter performance and mechanical hysteresis at 0°C, +30°C and +60°C is used to evaluate wet traction, dry traction and rolling resistance, respectively.

Table 7 below shows a general summary of the dynamic properties of the rubber tyre tread composition of this example, comparing the use of GFE as a process oil as an alternative to MES.

**TABLE 7**

| DYNAMIC MECHANICAL ANALYSIS | | | |
|---|---|---|---|
| COMPONENT | UNIT | GFE | MES |
| Elastic modulus -20°C | Mpa | 60,5 | 81,0 |
| Elastic modulus 0°C | Mpa | 37,2 | 43,2 |
| Elastic modulus +30 °C | Mpa | 25,2 | 25,7 |
| Elastic modulus +60 °C | Mpa | 17,7 | 17,7 |
| Viscous modulus at -20°C | Mpa | 10,7 | 22,6 |
| Viscous modulus at 0°C | Mpa | 5,2 | 7,5 |
| Viscous modulus at +30 °C | Mpa | 2,7 | 3,10 |
| Viscous module at +60 °C | Mpa | 2,0 | 1,6 |
| Tan δ (E"/E' ratio). at -20°C | - | 0,18 | 0,28 |
| Tan δ (E"/E' ratio). at 0°C | - | 0,14 | 0,17 |
| Tan δ (E"/E' ratio). and + 30°C | - | 0,11 | 0,12 |
| Tan δ (E"/E' ratio). and + 60°C | - | 0,11 | 0,09 |
| Peak value of Tan δ | - | 0,39 | 0,37 |
| Glass transition temperature | °C | -75 | -68 |
| Peak temperature E" | °C | -65 | -55 |
| Tan δ Peak Temperature | °C | -51 | -38 |

Comparison of DMA data shows that GFE is an excellent stretch agent for winter tires, as the glass transition temperature is -75°C, much lower than that of MES, which is -68°C and tan δ (E"/E' ratio) at -20°C is 0.18, which is also significantly lower than that of MES which is 0.28. Data from tan δ at 0°C to assess wet traction indicate that GFE, with tan δ = 0.14, provides better results than MES, with tan δ = 0.17. Data from tan δ at 30°C to assess dry tensile strength indicate that GFE with tan δ = 0.11, provides comparable results compared to MES, with tan δ = 0.12. Data from tan δ at 60°C to assess rolling resistance indicate that GFE with tan δ = 0.11, provides lower results than MES, with tan δ = 0.09.

### Abrasion loss

Abrasion resistance has been measured according to ISO 4649.2017 Tests show a loss of 127 mm³ for GFE compared to 122 mm³ for MES. The loss for GFE is slightly higher than that for MES and in percentage terms the difference between the two values is modest, about 4%. Abrasion resistance is strongly influenced by the modulus of elasticity: the lower the elastic modulus, the higher the deformation is consequent on the greater the dispersion of energy due to the internal friction between the molecules, proportional to the stretching of the polymer chains. However, it must be borne in mind that the comparison was made with the same composition. As explained below, increasing the degree of cross-linking can improve abrasion resistance while maintaining good elasticity properties.

### Conclusions

The results of the comparison tests with MES oils carried out on elastomer compounds with the typical compositions used for tyre treads indicate that, with the same dosages in the compositions, GFE significantly improves the road holding performance at low temperatures, while there is a worsening of the parameters that provide indications on rolling and abrasion resistance, which in the latter case is of modest magnitude.

In percentage terms, the loss of rolling resistance is 22%, which means that there is a slight increase of 2-3% in energy consumption. On the other hand, an increase in the elasticity characteristics of the elastomer inevitably leads to an increase in elastic energy losses because the thermal dissipation energy is proportional to the square of the elongation; with the same effort, the elongation increases proportionally as the modulus of elasticity decreases. However, a slight increase in energy consumption amply justifies the increased safety achieved by using tyres containing plasticisers that provide adequate flexibility for the tyre in low temperature conditions. Even the slight increase in tyre wear due to abrasion is largely offset by the increased driving safety.

It should be considered that the comparison between GFE and ESM was made with the same formulation. Therefore, a change in the concentrations of process oil, filler or vulcanizing agent leads to a consequent change in the performance parameters. It is observed that with GFE there is a reduction in torque and this means that there has been less cross-linking probably due to a partial subtraction of sulfur by GFE. Therefore, by increasing, for example, the dosage of sulphur and accelerating agents, the degree of cross-linking of the compound can be increased, which leads to an increase in the modulus of elasticity and consequently in rolling and abrasion resistance.

With the formulation according to the present invention, GFE gives an elongation at break after aging of 316%, while MES provides an elongation at break of only 216%; the improvement is remarkable, equal to 46%. In addition, the tensile strength was found to be 12.8 MPa for GFE and 10.4 MPa for MES. These data are influenced by the elasticizer's ability to facilitate the unwinding of polymer chains, resulting in more effective distribution and orientation of the chains, and by the dispersion capacity of the charges, carbon black and/or silica. If GFE confers higher values on elongation and tensile strength at break, this means that the increase in the degree of cross-linking is very likely to result in a substantial improvement in rolling and abrasion resistance without excessively penalizing winter performance.

### EXAMPLE 2. NBR elastomers, comparison with aromatic oil MES

Acrylonitrile butadiene rubber (NBR) is an unsaturated statistical copolymer of acrylonitrile and butadiene. NBR applications vary depending on the acrylonitrile content and molecular weight of the polymer. The polarity characteristics of NBR are provided by the acrylonitrile component. The higher the fraction of acrylonitrile, the higher the polarity of the polymer.

NBR elastomers are widely used in the automotive and petroleum industries for engine oil and fuel transportation equipment, machinery, pumps, and so on, but the largest use of NBR is in the production of medical gloves.

Nitrile rubber, which has always been recognized as a highly durable glove compound, is more durable than vinyl gloves and thus growing in popularity. Nitrile has been around for a while, but it has only become more affordable recently; thus, it is gaining popularity in medical, food, cleaning, and other industries. A few of the important features that nitrile gloves offer are their higher resistance to puncture than any other glove material. Additionally, nitrile rubber gloves fit snugly and make the wearer feel comfortable while working. Nitrile gloves are used as a precaution against allergy to natural rubber. This makes them suitable for medical applications in work environments where contact with body fluids, micro-organisms, and chemicals is possible. Other industries that use nitrile gloves include automotive, general industry, carpentry, roofing, and others. They generally use gloves that are coated with a durable, grippy nitrile coating, which offers chemical and water resistance.

Table 8 below shows a typical composition used for the production of elastomers based on nitrile rubber. In the second column from the left, the reference oil is GFE obtained from soy oil, while in the third the MES aromatic oil. The percentage amounts, measured in parts per 100 parts of polymer as it is (phr) are the same for both.

**TABLE 8**

| COMPOSITION FOR NITRILE RUBBER | | |
|---|---|---|
| COMPONENT | GFE (PHR) | MES (PHR) |
| NBR 34% acrylonitrile | 100 | 100 |
| Carbon Black N375 | 40 | 40 |
| Process oil (GFE, MES) | 20 | 20 |
| Cross-linking agent sulphur | 2.0 | 2.0 |
| Zinc oxide | 2.8 | 2.8 |
| Cyclohexyl-benzothiazole-sulfonamide | 1,0 | 1,0 |
| Antioxidant | 1,0 | 1,0 |

The compounds were prepared using a two-stage mixing procedure in a laboratory mixer. At first, the polymer was introduced, and soon after, processing oil, carbon black, stearic and all other components were added, except for sulphur and accelerator and CBS, which were added after 5 minutes. The tensile and tear strength of the samples was determined according to ISO 37:2017 and ISO 34-1:2015. The hardness measurement Hardness (Shore A) was determined according to DIN ISO 7619-1:2009.

The Shore A hardness tester is a device for measuring the hardness of polymers. Higher numbers on the Shore A scale indicate greater resistance to penetration by a special foot and therefore harder materials. Lower numbers indicate less strength and softer materials. The compression set (CS) values were determined in accordance with DIN ISO 815-1 2020. The aging test was carried out by keeping the samples for 500 hours at 60°C and at the end of the treatment, tensile strength, hardness, compression measurements were carried out again to evaluate the variations.

Table 9 below shows the rheometric data. MH is the torque after 10 minutes of vulcanization at 175 °C.

**TABLE 9 - TORQUE**

| Process oil | TORQUE Nm |
|---|---|
| No additions | 1,2 |
| GFE | 0,7 |
| MES | 0,6 |

Table 10 below shows the values of tensile strength, elongation at break, tear strength and Shore A hardness.

**TABLE 10 - MECHANICAL PERFORMANCE**

| Parameter | GFE | MES |
|---|---|---|
| Tensile Strength (MPa) | 16 | 13 |
| Strain at Break (%) | 590 | 480 |
| Tear strength (N/mm) | 9,8 | 7,3 |
| Shore A hardness (num) | 53 | 50 |
| Compression set (%) | 10 | 8 |

As can be seen from Table 10, the replacement of MES with GFE leads to a 23% improvement in tensile strength, 23% improvement in strain at break, and 34% improvement in tear strength.

Table 11 below shows the results of the mechanical properties of GFE after aging for 500 hours at 60 °C. As can be seen, GFE shows better preservation of mechanical properties even after aging.

**TABLE 11**

| DATA AFTER THERMO-OXIDATIVE AGING AT 60°C 500 HOURS | | |
|---|---|---|
| PARAMETER | GFE | MES |
| Tensile Strength (MPa) | 15 | 12,5 |
| Compression set (%) | 8 | 6 |
| Shore A hardness (num) | 60 | 60 |

The results show that after thermo-oxidative aging, the performance properties, if we exclude Shore A Hardness, undergo a modest and insignificant deterioration.

### EXAMPLE 3. NBR elastomers, comparison with dicarboxylic acid esters

In this example, the difference in tire performance is compared by replacing a bicarboxylic ester process oil with the GFE ester of the present invention. GFE made from soy oil was used, while Dioctyl Adipate (DOA) and Dibutyl Sebacate (DBS) were chosen as typical reference esters plasticizers. To this end, a typical formulation for oil resistant conveyor belt, shown in Table 12, was subjected to experimental tests. This formulation is based on a mixture of following elastomers: a) KUMHO KNB 40M, characterized by 41% Acrylonitrile (ACN) content and Mooney viscosity of 60; it is designed for a wide range of technical articles requiring good processability and very high oil and fuel resistance; b) Europrene N OZO 7028, which is a blend 70/30 NBR/PVC and the NBR is characterized by an ACN content of 19.5%. The Mooney viscosity is 75. It is characterised by an excellent processability, high resistance to oils and outstanding fire resistance. This grade represents the best compromise between ozone resistance and low temperature performances. The filler is Carbon Black N772. The other components are all typical elastomer vulcanization additives with the composition of this example. Relative quantities are expressed in parts by weight per 100 parts of total elastomers (phr). For example, the plasticizers, each have a concentration of 30 parts referred to 100 parts by weight of total elastomers. In Table 12, the samples have been initialed with the acronym of the extender contained in it.

**TABLE 12**

| COMPOSITION FOR NITRILE RUBBER | | | |
|---|---|---|---|
| COMPONENT | DBS (phr) | DOA (phr) | GFE (phr) |
| NBR KUMHO KNB 40M | 50 | 50 | 50 |
| Europrene N OZO 7028 | 50 | 50 | 50 |
| Carbon Black N772 | 45 | 45 | 45 |
| ZnO | 5 | 5 | 5 |
| Dibutyl Sebacate (DBS) | 30 | | |
| Dioctyl Adipate (DOA) | | 30 | |
| GFE | | | 30 |
| TMTM | 0,3 | 0,3 | 0,3 |
| CBS | 1,5 | 1,5 | 1,5 |
| Sulfur | 1,8 | 1,8 | 1,8 |
| Retarder PVI | 0,3 | 0,3 | 0,3 |

### Preparation of rubber composition

Two step-by-step methods were used in a 1.5-litre mixer to prepare the composition of the rubber to be tested. In the first phase, elastomers, fillers and plasticizers are introduced into the mixer and the mixture is energetically mixed at temperatures between 150°C and 180°C in order to optimize the distribution of the filler in the mixture of the elastomer chains. In the second step, the elastomer cross-linking reaction is performed. The mixture is cooled to temperatures below 110 °C and then the necessary components for cross-linking are introduced.

### Test methods

The rheometric curves were recorded on an oscillating disc rheometer (ODR) at 175°C for 6 minutes according to the general rules of ISO 3417:2008. The stress-strain properties and final properties of the elastomer specimens were tested according to the ISO 37:2017 test method. Aged stress-strain properties were determined on elastomer specimens aged in the furnace for 3 days at 100°C. The measurement of Shore A Hardness was performed according to ISO 7619-1:2010.

Table 13 shows some characteristic data measured by the rheometer during the vulcanization process of the elastomer whose composition is shown in table 12. MH represents the maximum torque reached by the rheometric curve at the end of the cross-linking reaction, while ML is the minimum torque before starting vulcanization.

**TABLE 13. RHEOMETRIC TEST - TORQUE DATA**

| Torque 175°C x 6 min | DBS | DOA | GFE |
|---|---|---|---|
| ML (dN m) | 3,88 | 3,71 | 4,43 |
| MH (dN m) | 45,60 | 37,50 | 41,79 |
| MH-ML (dN m) | 41,72 | 33,79 | 37,36 |

ML provides an indication of the viscosity of the uncured rubber compound, and there is no evidence of significant differences between the GFE oil and the reference DBS e DOA esters. MH is correlated with the modulus of elasticity at the end of crosslinking. MH-ML is correlated with cross-linking density and the lowest value is that of DOA, but there is little difference with GFE (about 10%).

### Hardness

The hardness of elastomers is measured by the Shore A hardness tester. Higher numbers on the Shore A Hardness scale indicate greater resistance to penetration by a special foot and therefore harder materials. Lower numbers indicate less strength and softer materials.

As can be seen from the table 14 below, hardness of rubber with GFE is equivalent to references before aging and after ageing the hardness of GFE undergoes an increment analogous to DOA.

**TABLE 14**

| SHORE A HARDNESS | | | |
|---|---|---|---|
| Shore A hardness | DBS | DOA | GFE |
| After cross-linking | 61 | 63 | 62 |
| Maturation 3 days at 100 °C | 70 | 64 | 65 |

### Mechanical properties after cross-linking

Table 15 shows modulus, tensile strength, and elongation at break after crosslinking.

**TABLE 15**

| STRESS-STRAIN PROPERTIES AFTER CROSS-LINKING | | | | |
|---|---|---|---|---|
| | Unit | DBS | DOA | GFE |
| Modulus at 50% elongation, | Mpa | 1,4 | 1,3 | 1,2 |
| Modulus at 100% elongation | Mpa | 2,4 | 2,3 | 2,0 |
| Modulus at 200% elongation | Mpa | 4,7 | 4,6 | 3,8 |
| Modulus at 300% elongation | Mpa | 7 | 7,1 | 5,6 |
| Tensile strength at break | Mpa | 11,1 | 11,8 | 11,9 |
| Elongation at break | % | 460 | 474 | 730 |

In general, elastomers with low modulus give greater elongation and lower tensile strength, while rubber with GFE as a process oil shows a high elongation at break, +54% compared to DOA with a tensile strength at break, a little better respect to DOA and DBS.

### Mechanical properties after aging

Table 16 shows modulus at different degrees of elongation, stress and the elongation at break after 3-day aging of elastomer specimens at 100°C

**TABLE 16**

| STRESS-STRAIN PROPERTIES AFTER AGING | | | | |
|---|---|---|---|---|
| Tensile strength | Unit | DBS | DOA | GFE |
| Effort at 50% elongation, | Mpa | 2,6 | 1,7 | 1,0 |
| Modulus at 100% elongation | Mpa | 4,6 | 3,0 | 1,8 |
| Modulus at 200% elongation | Mpa | 9,3 | 6,6 | 3,6 |
| Modulus at 300% elongation | Mpa | 13,7 | 10,8 | 5,5 |
| Stress at break | Mpa | 14,3 | 11,5 | 10,9 |
| Elongation at break | % | 314 | 320 | 568 |

Also After aging, GFE manages to maintain lower Young's modulus and higher elongations than DBS and DOA. The elongation at break of GFE compared to DOA is 77% greater, an even better figure than the values obtained on unaged elastomers. Tensile strength at break is slightly lower than DOA, but this is of little importance, because the energy consumed for breaking is equivalent to Work = force x displacement and GFE has elongations much more greater compared to the other two extenders even after aging

### EXEMPLE 4

### Nitrile and latex gloves

GFE esters can be introduced among the components of medical gloves when it is desired to increase its flexibility, to better disperse fillers such as calcium carbonate, to decrease permeability to chemicals or to pathogenic organisms, towards which they have a destructive action.

However, the mixing of GFE with elastomer involves a change in the mechanical characteristics of the material that makes up the glove, and it is necessary to verify that the tensile strength remains within the limits of acceptance.

In this example, a hand-shaped ceramic mold in which a nitrile or latex medical glove has been inserted has been immersed for 1 minute in a container filled with the GFE ester. It was then removed and placed in an oven kept at 60°C for a period of 30 minutes. The mold (called "dipping former" in industrial practice) was left hanging from the ceiling of the oven with a hook in order to allow the liquid that was not absorbed by the glove to drip out. At the end of the treatment the gloves were weighed.

The esters subjected to the treatment were two: an ester as it is obtained from the esterification of Glycerol Formal with soy fatty acids, called GFE-A and the same ester where the double bonds have been partially epoxidized, called GFE-B. The gloves appeared dry and there were no indications of surface oiliness, showing that the ester was absorbed by the polymer material. The extent of GFE absorption was assessed by measuring the difference in glove weight before and after treatment.

The values are shown in the below table 17. On the basis of the weight increase of the gloves, it can be observed that under the same conditions in the treatment (immersion and stove maintenance times), it appears that the GFE-B sample has a better absorption capacity in nitrile, while GFE-A is better absorbed in latex.

**TABLE 17**

| Glove weight change after absorption | | | | |
|---|---|---|---|---|
| | Nitrile | | Latex | |
| | GFE A | GFE B | GFE A | GFE B |
| Initial weight, gr | 3,230 | 3,333 | 6,065 | 6,069 |
| Final weight, gr | 3,940 | 4,166 | 6,914 | 7,040 |
| Weight gain, % | 22 | 31 | 14 | 11 |

The break force of samples obtained from gloves was measured according to the methods indicated in paragraph 5.2 of the EN 455-2-2015 standard: "Medical gloves for single uses- Requirements and testing for physical properties". It has been prepared a dumb-bell test piece using a cutter as specified above from the palm, avoiding textured areas if possible and taking the test pieces in the direction of the longitudinal axis of the glove.

**TABLE 18**

| Mechanical parameters after GFE absorption by immersion | | | | |
|---|---|---|---|---|
| | GFE A | GFE B | Nitrile GFE A | Latex GFE B |
| Tensile strength (MPa) | 26 | 24 | 16 | 15 |
| Elongation at break % | 560 | 530 | 670 | 650 |

It can be seen that the introduction of Glycerol Formal fatty acid esters in the amounts indicated in the elastomers in this example, the tensile strength remains above the minimum acceptance limit of the ISO D3578 2002 standard for gloves for examination or procedure gloves, which is 14 Mpa.

From this example it emerges, on the basis of the amount of ester absorbed at the same absorption time, that GFE esters whose double bonds are partially epoxidized, show greater compatibility in polar elastomers such as NBR.

### EXEMPLE 5

### Permeability test on natural rubber

To evaluate the resistance to virus penetration of nitrile and latex gloves with GFE additives, we used the φX174 bacteriophage penetration test described in the publication "PCR-Based Method for Detecting Viral Penetration of Medical Exam Gloves" (J Clin Microbiol. 2002 Aug;40(8):2725-8. Broyles, O' Connell, Korniewicz). A latex glove as it was just taken out of the package was compared with the latex gloves in the previous example which have been added with the plasticizers named GFE-A and GFE-B.

The methods of carrying out the test have been described in the chapter. "Materials and methods" of the above publication, which is reported below.

A polypropylene beaker was filled with 200 ml of sterile nanopure water. For proof-of-principle testing, each finger of the gloves was punctured five times with an 18-gauge needle and then held over the water-filled beaker and filled with a solution containing bacteriophage (10⁵ to 10⁶ PFU/ml in sterile H₂O), ensuring that all five fingers were filled. The glove was placed into the water-filled beaker and left at room temperature for 30 min, after which samples were drawn from inside and outside the glove. Samples were serially diluted in 10-fold increments and tested for the presence of bacteriophage by coculture in top agar with the bacterial host. After diluted samples (100 µl) were mixed with E. *coli* and the top agar and poured onto NB medium plates, the plates were incubated for 3 to 4 h at 37°C and the plaques were counted.

In our case, we prepared a solution containing bacteriophage at a concentration of around 10⁸ PFU/ml, in order to make the differences more marked. The following table 19 shows the results of the penetration of punctured and latex gloves by bacteriophage

**TABLE 19**

| Results on latex permeability | | |
|---|---|---|
| | Conc. inner PFU/ml | Conc. Outer PFU/ml |
| Control | 13.500 ×10⁴ | 112 ×10⁴ |
| With GFE-A | 13.500 ×10⁴ | 27 ×10⁴ |
| With GFE-B | 13.500 ×10⁴ | 18 ×10⁴ |

It can be seen that both GFE esters allow the penetration of the virus through the material to be completely cancelled. This property of making the elastomer totally waterproof is very important in the production of condoms, as it allows to guarantee absolute protection of the user from viruses and sexual bacteria, in particular AIDS.

## Claims

1. Use of C₈-C₁₈ fatty acid esters of Glycerol Formal as a process oil for the production of synthetic elastomers.

2. Use of esters according to claim 1, in which fatty acids are chosen from caprylic, lauric, myristic, stearic, palmitic, oleic, linoleic, linolenic, ricinoleic and their mixtures.

3. Use of esters according to claims 1 and 2 where elastomers are chosen from styrene butadiene rubber (SBR), nitrile butadiene rubber (NBR), butadiene rubber (BR), natural rubber (NR), isoprene rubber (IR), polyurethane rubber and mixtures thereof.

4. Use of esters according to claim 1 to 3, wherein double bonds are partially epoxidized for the production of synthetic polar elastomers as nitrile and polyurethane rubbers.

5. Use of esters according to claims 1 and 3 for the production of tire tread capable of giving the elastomer a glass transition temperature Tg of less than -74°C and a viscous modulus at -20°C of less than 11 MPa.

6. Use of esters according to claim 1 to 3 and 5 in elastomeric compositions for the production of automotive tyre treads, wherein said compositions comprises 20 to 90% wt of functionalised styrene-butadiene rubber (SBR), optionally and 10 to 80% of at least one rubber to be chosen from polybutadiene rubber (BR), natural rubber (NR), polyisoprene rubber (IR) and their mixtures.

7. Use di esters according to claim 1 and 4 in elastomeric compositions for the production of medical devices as condom and medical gloves.

8. Elastomeric composition for tires comprising elastomers in quantities between 20% and 90% by weight, and at least one formal ester of C₈-C₁₈ fatty acids of glycerol in quantities between 10% and 80% by weight.

9. Elastomeric composition according to claim 8, wherein the tensile strength at break is greater than 11.00 Mpa, preferably greater than 12.00 Mpa, after aging.

10. Tire tread comprising the elastomeric composition according to claim 6 or 7, wherein the glass transition temperature is less than -70°C, the ratio of E"/E' at - 20°C is less than 0, 2, the ratio between E"/E' at 0°C is less than 0.15.

11. Elastomeric composition for condoms and medical gloves comprising elastomers in quantities form 99% to 40% and from 1% to 60 % wt of formal ester of C₈-C₁₈ fatty acids of glycerol.
